(19) Europäisches Patentamt
European Patent Office
Office européen des brevets

(11) Publication number: **0 153 404**
**B1**

(12) # EUROPEAN PATENT SPECIFICATION

(45) Date of publication of patent specification: **13.01.88**

(51) Int. Cl.$^4$: **C 08 K 9/04,** C 08 K 5/54

(21) Application number: **84903313.9**

(22) Date of filing: **21.08.84**

(86) International application number:
**PCT/US84/01341**

(87) International publication number:
**WO 85/01055 14.03.85 Gazette 85/07**

(54) **THIXOTROPIC CYANOACRYLATE COMPOSITIONS.**

(30) Priority: **31.08.83 US 528275**

(43) Date of publication of application:
**04.09.85 Bulletin 85/36**

(45) Publication of the grant of the patent:
**13.01.88 Bulletin 88/02**

(84) Designated Contracting States:
**DE FR GB**

(56) References cited:
**US-A-3 607 542**
**US-A-3 663 501**
**US-A-3 839 065**
**US-A-3 896 077**
**US-A-3 940 362**
**US-A-4 076 685**
**US-A-4 102 945**
**US-A-4 180 911**
**US-A-4 180 913**
**US-A-4 320 047**

(73) Proprietor: **LOCTITE CORPORATION**
**705 North Mountain Road**
**Newington, Connecticut 06111 (US)**

(72) Inventor: **LITKE, Alan, Edward**
**155 N. Hoadley Street**
**Naugatuck, CT 06770 (US)**

(74) Representative: **Marchant, James Ian et al**
**Elkington and Fife High Holborn House 52/54**
**High Holborn**
**London WC1V 6SH (GB)**

**EP 0 153 404 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

### Background of the invention

The present invention relates to compositions comprising an α-cyanoacrylate and a thixotropic agent.

Cyanoacrylate adhesives based on esters of α-cyanoacrylic acid have gained wide acceptance in recent years for a broad range of industrial consumer uses. The cyanoacrylate esters themselves, however, are very low viscosity liquids which makes the use of unfilled compositions difficult. Such compositions often migrate from the bondline or are absorbed into porous surfaces. Accordingly, there have been developed a variety of thickened cyanoacrylate adhesive compositions which incorporate organic polymers and/or inorganic fillers to reduce adhesive migration. Examples of such compositions are described in U.S. Patents 3,607,542; 3,896,077, 4,012,840, 4,105,715, 4,180,913 and in Chemical Abstracts *89*:117907c; *89*:216475u; *91*:40425c; and 92:95114b.

In the art of thickened cyanoacrylates it is sometimes desirable that the composition display thixotropic properties. Thus, under high shear conditions the composition can be processed and easily applied to the substrate but once applied, will display significantly reduced migratory tendencies. It is also well known that the inclusion of fumed silicas in many organic liquid compositions produces thixotropic effects. The use of such silicas has been reported in cyanoacrylate compositions.

At least certain of the prior art cyanoacrylate compositions employing fumed silicas have displayed stability problems, however. Thus U.S. Patent 3,607,542 describes organically filled cyanoacrylate compositions in which fumed silica is an optional ingredient. These compositions are reportedly stable for only up to 4 hours. Furthermore, the same patent states that silica by itself does not readily mix with cyanoacrylate monomer to form a paste.

Cyanoacrylate formulations also occasionally find non-adhesive applications. Thus, for instance, the vapors of methyl and ethyl cyanoacrylates have found use for developing latent fingerprints in law enforcement applications. For such applications it would be desirable to develop a nonflowable form of cyanoacrylate monomer so that small open containers of the monomer can be placed throughout a room or automobile to release vapors without the danger of accidental monomer spillage. Desirably the thixotropic additives will also be kept to a minimum so as not to substantially reduce the monomer vapor pressure.

In certain applications it has been discovered that hydrophobic silicas produced by treatment of fumed silica with dimethyldichlorosilane can be used to give thixotropic cyanoacrylate compositions with improved stability. However, these fillers add cure retarding strong acid to the cyanoacrylate composition. Also, the thixotropic ratio of these silicas in cyanoacrylate esters (the ratio of apparent viscosity is measured under specified high and low shear conditions) is quite low. Therefore, when very high thixotropic effects are desired, such as when a pasty composition is desired, the amount of acid introduced by the silica can substantially reduce the cure time of the composition. Also, for latent fingerprint developing type applications, higher silica levels may result in lower monomer vapor pressures.

Commercially available fumed silicas are also known which have been treated with hexamethyldisilazine. It has been discovered that thixotropy ratios are also very low for these materials and that at least some of these materials tend to destabilize cyanoacrylate compositions. The destabilization effect is thought to result from residual ammonia or amine in the filler.

Accordingly there exists a need for a cyanoacrylate thixotrope, stable to the cyanoacrylate monomer, which has a significantly improved thixotropy ratio over dimethyldichlorosilane or hexamethyldisilazine treated silicas, and which does not adversely affect the fixture time of adhesive formulations.

### Summary of the invention

The present application relates to cyanoacrylate compositions which employ fumed silicas treated with a polydimethylsiloxane or a trialkoxyalkylsilane as a thixotropic additive. It has been unexpectedly found that such silicas when incorporated into cyanoacrylate compositions do not adversely effect the stability of the composition, display a significantly higher thixotropy ratio than the previously mentioned treated silicas and, in adhesive compositions, do not adversely effect fixture time even at levels of about 10—12% where the compositions become pasty and very difficult to stir or apply uniformly.

The inventive compositions may consist primarily of an appropriately stabilized cyanoacrylate ester monomer and the specified silica at a level of between about 0.5 and 12% based on total composition weight. However, it is often preferred that a small amount of an organic polymer such as polymethylmethacrylate be dissolved in the monomer.

A preferred embodiment of the invention is a cyanoacrylate monomer formulation as described above in the form of a nonflowable gel. As used herein a nonflowable gel is one which will not move when extruded onto a vertical glass plate as a 3.2 mm (1/8") diameter bead.

The inventive compositions are also useful in nonadhesive applications such as nonflowable latent fingerprint developing formulations.

### Detailed description of the invention

The α-cyanoacrylate monomers used in the inventive compositions are compounds of the formula:

$$\begin{array}{c} \quad CN \;\; O \\ \quad | \quad \| \\ CH_2{=}C{-}C{-}OR^1 \end{array}$$

wherein $R^1$ represents a straight chain or branched chain alkyl group having 1 to 12 carbon atoms (which may be substituted with a substituent such as a halogen atom or an alkoxy group) a straight chain or branched chain alkenyl group having 2 to 12 carbon atoms, a straight chain or branched chain alkynyl group having 2 to 12 carbon atoms, a cycloalkyl group, an aralkyl group or an aryl group. Specific examples of the groups for $R^1$ are a methyl group, an ethyl group, an n-propyl group, an isopropyl group, an n-butyl group, an isobutyl group, a pentyl group, a hexyl group, an allyl group, a methallyl group, a crotyl group, a propargyl group, a cyclohexyl group, a benzyl group, a phenyl group, a cresyl group, a 2-chloroethyl group, a 3-chloropropyl group, a 2-chlorobutyl group, a trifluoroethyl group, a 2-methoxyethyl group, a 3-methoxybutyl group and a 2-ethoxyethyl group. Most preferred are methyl, ethyl and 2-methoxyethyl cyanoacrylate.

Fumed silicas which have been found to impart desired thixotropic properties to the inventive compositions fall into two categories. The first, and most preferred, are polydimethylsiloxane treated silicas such as Cab-O-Sil N70-TS™, sold by the Cabot Corporation. The silica has a carbon content of 5 weight percent and a surface area of $70M^2$/gm according to the manufacturer. The product is the result of a process in which trimethylsilyl (TMS) terminated silicone fluid is absorbed onto the fumed silica and treated at elevated temperatures (about 200°C). Some of the silanol groups on the silica are replaced by TMS terminated polydimethylsiloxane chains. Substantial silanol content, however, remains on the particle and the silicone fluid may be present predominantly as adsorbed silicone rather than as covalently bonded polymer fragments. An equivalent product is believed to be Aerosil R202 sold by Degussa.

The combination of stability and high thixotropy of the inventive compositions containing polydimethyl-siloxane treated silicas is especially surprising because these silicas retained substantial amounts of surface silanol groups and because they have relatively low surface areas. Heretofor it had been observed that shelf stability was correlated with the degree of surface treatment so that silicas having the highest amounts of surface silanol generally gave the poorest shelf stability. Additionally, as is the case with other materials, high surface treatment gave much poorer thixotropic properties, and depending on the particular surface treatment, may not improve the shelf stability. Furthermore, low surface areas also correlate with poor thixotropic properties.

The second category of silicas usable in the inventive compositions are trialkoxy alkyl silanes. An example is Aerosil R805™, available from Degussa Corporation Aerosil R805™ is a trimethoxyoctylsilane treated silica having a surface area of 150 $m^2$/gm.

The superior thixotropic performance of the inventive cyanoacrylate compositions is best obtained at silica levels of about 4—8%. These properties were demonstrated by comparative testing of an ethyl cyanoacrylate formulation containing 6% of a .4—.5 million mw polymethylmethacrylate and 6% hydrophobic silica. The composition was stabilized with 5 ppm methanesulfonic acid (MSA) approximately 2500 ppm hydroquinone and 5 ppm $SO_2$. The formulations were prepared with the aforementioned Cab-O-Sil N70-TS™ and Aerosil R805™ as well as 2-hexamethyldisilazine treated silicas (Tellenox 500™ sold by Tulco, Inc. and Wacker HDK 2000™ sold by Wacker-Chemie) and 3-dimethyldichlorosilane treated silicas (Aerosils R972™, R974™ and R976™, all sold by Degussa). Table I gives the results of comparative Brookfield viscosity at 21°C (70°F) and acid level (calculated as ppm MSA) determinations. Brookfield viscosity is equivalent to mPa · s.

TABLE I

| | | Brookfield viscosity (equivalent to mPa · s) | | | |
|---|---|---|---|---|---|
| Comp. | Silica | 2.5 RPM helipath (spindle) | 20 RPM helipath (spindle) | Ratio 2.5/20 | Total acid |
| A | Cab-O-Sil N70-TS™ | $2.9\times10^5$ (TE) | $4.2\times10^4$ (TE) | 6.9 | 27 |
| B | Aerosil R805™ | $1.4\times10^5$ (TE) | $2.8\times10^4$ (TE) | 5.0 | 32 |
| C | Tellenox 500™ | $4.4\times10^4$ (TE) | $2.1\times10^4$ (TE) | 2.1 | 26 |
| D | Wacker HDK2000™ | $3.3\times10^2$ (TA) | $3.0\times10^2$ (TA) | 1.1 | 29 |
| E | Aerosil R972™ | $1.2\times10^4$ (TC) | $3.8\times10^3$ (TC) | 3.2 | 38 |
| F | Aerosil R974™ | $2.1\times10^4$ (TC) | $5.2\times10^3$ (TC) | 4.0 | 40 |
| G | Aerosil R976™ | $2.6\times10^4$ (TC) | $8.1\times10^3$ (TC) | 3.2 | 39 |

As can be seen from the table, compositions A and B, which are within the invention, show higher low shear viscosity and substantially higher thixotropic ratios than compositions C—G which are not within the invention. It was also observed that, when unagitated, compositions A and B were nonflowable gels whereas compositions C—G were all ungelled and pourable.

The significantly higher acid numbers of compositions E—G is evidence that the dichlorosilane treated silicas do contribute strong acid to the cyanoacrylate formulations.

Both hexamethyldisilazine treated silicas had impractically low thixotropic ratios as shown by Table I. Furthermore, at least one of the hexamethyldisilazane treated silicas appears to destabilize cyanoacrylate monomers. Formulation C, which utilizes the Tellenox 500™ silica polymerized in less than one day in a sealed tube at 82°C while compositions A, B and D—G all lasted at least 15 days under the same conditions.

When a sample of a commercial polymer thickened ethyl cyanoacrylate adhesive, Loctite® Superbonder® 416, a product of Loctite Corporation, Newington, Connecticut, was compared for fixture speed, speed of cure, hot strength, heat aging, humidity resistance, and thermal cycling resistance, with a sample as in composition A except that stabilizer levels were 10 ppm MSA, 1000 ppm hydroquinone and 10 ppm $SO_2$, substantially equivalent results were obtained. Storage stability of the inventive product was in excess of 8 days in a glass tube at 82°C. The viscosity of the Superbonder® product was about 1600 mPa · s (cps) whereas the inventive product was a thixotropic gel as in composition A.

It is generally preferred that the cyanoacrylate compositions of the invention include a minor amount of dissolved organic polymer. Suitable polymers include polyacrylates and polymethacrylates, polycyanoacrylates such as poly(ethylcyanoacrylate), and poly(vinyl acetate) polymers and copolymers. The organic polymers are preferably included within the range of approximately 1—15% of the composition by weight. Preferably, the organic polymers are included in the range of 3—10%. The inclusion of the organic polymer is recommended in order to prevent or significantly diminish the settling out of the silica from the inventive compositions. The compositions containing dissolved polymer are also observed to produce higher viscosities at equivalent silica concentrations and to recover thixotropic behavior faster after agitation than without dissolved polymers.

Other additives, conventional within the cyanoacrylate formulation art, may be included within the compositions of the invention without departing from the teaching hereof. Examples of such additives need not be specified since they are within the skill of those working in the art. See, e.g., U.S. Patent 4,170,585, and 4,450,265.

As adhesives, the stable gel formulations of the invention are especially useful on porous substrates such as wood and ceramics which traditionally have been difficult to bond, in part because of adhesive migration from the bondline before cure. The gel holds the adhesive in the bondline until cure. The gel adhesive also provides significant safety advantage because even the prior commercial thickened cyanoacrylate adhesives could penetrate clothing when spilled, occasionally causing skin burns as the spilled monomer polymerized.

The nonflowable gels of the invention will not penetrate ordinary clothing.

Gel adhesives for wood or other deactivating surfaces also preferably include about 0.1—2% of an accelerator compound such as silacrown or calixarene additives such as 1,1-dimethylsila-17-crown 6, 37,38,39, 40,41,42 hexa(2-ethoxy-2-oxo)ethyl calix [6]arene and 5,11,17,23-tetrat-butyl-25,26,27,28-tetra(2-ethoxy-2-oxo)ethyl calix [4]arene.

As mentioned above, it would be desirable for latent fingerprint developing applications to have a nonflowable form of cyanoacrylate with high vapor pressure. Gel compositions of the invention which have been stabilized to the point where they will not instantly polymerize on contact with moisture are especially useful for such applications. The gel form prevents spillage and the over-stabilization guards against bonding of fingers ("finger-stick") or other articles. A typical such formulation includes 88% methylcyanoacrylate stabilized with 0.2% methane sulfonic acid and 2500 ppm hydroquinone, 6% polymethylmethacrylate and 6% polydimethylsiloxane treated silica.

Gel formulations of the invention can also be used to support microfiber additives stable to the cyanoacrylate monomer, such as glass or untreated polyethylene fibers, which provide reinforced cured products. Because of the gel structure fibers can remain uniformly suspended in the composition for months. Thus, rapid curing cyanoacrylate monomer based molding and repair putty compounds are feasible.

## Claims

1. A composition comprising an α-cyanoacrylate ester monomer and a thixotropic agent, characterised in that it contains from 0.5 to 12% by weight of the composition of a fumed silica having a surface treated with a polydimethylsiloxane or trialkoxyalkylsilane as thixotropic agent.

2. A composition as claimed in claim 1, characterised in that an organic polymer is dissolved in the monomer.

3. A composition as claimed in claim 2, characterised in that the polymer is present at a level of between 1 and 15% by weight.

4. A composition as claimed in claim 3, characterised in that the polymer is present at a level of between 3 and 10% by weight.

5. A composition as claimed in any of claims 2 to 4, characterised in that the polymer is a polyvinyl acetate polymer or copolymer, a polyacrylate, a polymethacrylate or a polycyanoacrylate.

6. A composition as claimed in claim 5, characterised in that the polymer is polymethylmethacrylate.

7. A composition as claimed in any of claims 1 to 6, characterised in that it has the consistency of a nonflowable gel, which will not move when extruded onto a vertical glass plate as a 3.2 mm (1/8") diameter bead.

8. A composition as claimed in any of claims 1 to 7, characterised in that the silica is a polydimethylsiloxane treated silica and is present in an amount between 4 and 8% by weight.

9. A composition as claimed in any of claims 1 to 7, characterised in that the silica is a trimethoxyoctylsilane treated silica and is present in an amount of between 4 and 8% by weight.

10. A composition as claimed in any of claims 1 to 9, characterised in that the α-cyanoacrylate monomer is a compound of the formula:

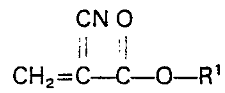

where $R^1$ is $C_{1-12}$ alkyl, $C_{1-12}$ alkyl substituted with a halogen atom or an alkoxy group, $C_{2-12}$ alkenyl, $C_{2-12}$ alkynyl, aralkyl or aryl.

11. A composition as claimed in claim 10, characterised in that $R^1$ is methyl, ethyl, n-propyl, isopropyl, n-butyl, isobutyl, pentyl, hexyl, allyl methallyl, crotyl, propargyl, cyclohexyl, benzyl, phenyl, cresyl, 2-chloroethyl, 3-chloropropyl, 2-chlorobutyl, trifluoroethyl, 2-methoxyethyl or 2-ethoxyethyl.

12. A composition as claimed in claim 10, characterised in that $R^1$ is methyl, ethyl or 2-methoxyethyl.

13. A method of bonding porous substrates characterised in that a composition according to any of claims 1 to 12 is applied to at least one of the substrates and the substrates are joined for sufficient time to permit the composition to cure.

**Patentansprüche**

1. Zusammensetzung, enthaltend ein α-Cyanoacrylatestermonomer und ein thixotropes Mittel, dadurch gekennzeichnet, dass sie 0,5 bis 12 Gew.-% der Zusammensetzung dampfbehandeltes Kieselgel mit einer mit einem Polydimethylsiloxan oder Trialkoxyalkylsilan behandelten Oberfläche als thixotropes Mittel enthält.

2. Zusammensetzung, wie in Anspruch 1 beansprucht, dadurch gekennzeichnet, dass ein organisches Polymer im Monomer gelöst ist.

3. Zusammensetzung, wie in Anspruch 2 beansprucht, dadurch gekennzeichnet, dass das Polymer in einer Menge zwischen 1 und 15 Gew.-% vorhanden ist.

4. Zusammensetzung, wie in Anspruch 3 beansprucht, dadurch gekennzeichnet, dass das Polymer in einer Menge zwischen 3 und 10 Gew.-% vorhanden ist.

5. Zusammensetzung, wie in einem der Ansprüche 2 bis 4 beansprucht, dadurch gekennzeichnet, dass das Polymer ein Polyvinylacetatpolymer oder -copolymer, ein Polyacrylat, ein Polymethacrylat oder ein Polycyanoacrylat ist.

6. Zusammensetzung, wie in Anspruch 5 beansprucht, dadurch gekennzeichnet, dass das Polymer Polymethylmethacrylat ist.

7. Zusammensetzung, wie in einem der Ansprüche 1 bis 6 beansprucht, dadurch gekennzeichnet, dass sie die Konsistenz eines nicht fliessfähigen Gels hat, welches sich bei Extrusion als Tropfen mit einem Durchmesser von 3,2 mm (1/8") auf eine senkrechte Glasplatte nicht bewegt.

8. Zusammensetzung, wie in einem der Ansprüche 1 bis 7 beansprucht, dadurch gekennzeichnet, dass das Kieselgel ein mit Polydimethylsiloxan behandeltes Kieselgel ist und in einer Menge zwischen 4 und 8 Gew.-% vorhanden ist.

9. Zusammensetzung, wie in einem der Ansprüche 1 bis 7 beansprucht, dadurch gekennzeichnet, dass das Kieselgel ein mit Trimethoxyoctylsilan behandeltes Kieselgel ist und in einer Menge zwischen 4 und 8 Gew.-% vorhanden ist.

10. Zusammensetzung, wie in einem der Ansprüche 1 bis 9 beansprucht, dadurch gekennzeichnet, dass das α-Cyanoacrylat-monomer eine Verbindung der Formel ist

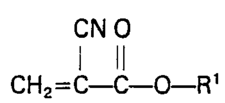

worin $R^1$ $C_{1-12}$-Alkyl, mit einem Halogenatom oder einer Alkoxygruppe substituiertes $C_{1-12}$-Alkyl, $C_{2-12}$-Alkenyl, $C_{2-12}$-Alkinyl, Aralkyl oder Aryl ist.

11. Zusammensetzung, wie in Anspruch 10 beansprucht, dadurch gekennzeichnet, dass $R^1$ Methyl, Ethyl, n-Propyl, Isopropyl, n-Butyl, Isobutyl, Pentyl, Hexyl, Allyl, Methallyl, Crotyl, Propargyl, Cyclohexyl,

Benzyl, Phenyl, Cresyl, 2-Chlorethyl, 3-Chlorpropyl, 2-Chlorbutyl, Trifluorethyl, 2-Methoxyethyl oder 2-Ethoxyethyl ist.

12. Zusammensetzung, wie in Anspruch 10 beansprucht, dadurch gekennzeichnet, dass $R^1$ Methyl, Ethyl oder Methoxymethyl ist.

13. Verfahren zum Verkleben poröser Substrate, dadurch gekennzeichnet, dass eine Zusammensetzung nach einem der Ansprüche 1 bis 12 auf wenigstens eines der Substrate angewandt wird und die Substrate für eine zum Aushärten der Zusammensetzung hinreichende Zeit zusammengefügt werden.

**Revendications**

1. Une composition comprenant un monomère ester α-cyanoacrylate et un agent thixotrope, caractérisée en ce qu'elle contient de 0,5 à 12% en poids, par rapport à la composition, d'une silice de fumée ayant une surface traitée par un polydiméthylsiloxane ou un trialcoxyalkylsilane comme agnet thixotrope.

2. Une composition selon la revendication 1, caractérisée en ce qu'un polymère organique est dissous dans le monomère.

3. Une composition selon la revendication 2, caractérisée en ce que le polymère est présent à un taux compris entre 1 et 15% en poids.

4. Une composition selon la revendication 3, caractérisée en ce que le polymère est présent à un taux compris entre 3 et 10% en poids.

5. Une composition selon l'une quelconque des revendications 2 à 4, caractérisée en ce que le polymère est un polymère ou copolymère acétate de polyvinyle, un polyacrylate, un polyméthacrylate ou un polycyanoacrylate.

6. Une composition selon la revendication 5, caractérisée en ce que le polymère est du poly (méthacrylate de méthyle).

7. Une composition selon l'une quelconque des revendications 1 à 6, caractérisée en ce qu'elle a la consistance d'un gel non coulable qui ne bougera pas lorsqu'extrudé sur une plaque de verre verticale sous la forme d'un cordon de 3,2 mm de diamètre.

8. Une composition selon l'une quelconque des revendications 1 à 7, caractérisée en ce que la silice est une silice traitée par du polydiméthylsiloxane et est présente en une quantité comprise entre 4 et 8% en poids.

9. Une composition selon l'une quelconque des revendications 1 à 7, caractérisée en ce que la silice est une silice traitée par du triméthoxyoctylsilane et est présente en une quantité comprise entre 4 et 8% en poids.

10. Une composition selon l'une quelconque des revendications 1 à 9, caractérisée en ce que le nonomère α-cyanoacrylate est un composé de la formule:

$$\begin{array}{l} \qquad\quad CN\ \ O \\ \qquad\quad \|\quad \| \\ CH_2=C-C-O-R^1 \end{array}$$

où $R^1$ est un radical alkyle en $C_{1-12}$, alkyle en $C_{1-12}$ substitué par un atome d'halogénure ou un groupe alcoxy, alcényle en $C_{2-12}$, aralkyle ou aryle.

11. Une composition selon la revendication 10, caractérisée en ce que $R^1$ est un radical méthyle, éthyle, n-propyle, isopropyle, n-butyle, isobutyle, pentyle, hexyle, allyle, méthallyle, crotyle, propargyle, cyclohexyle, benzyle, phényle, crésyle, 2-chloroéthyle, 3-chloropropyle, 2-chlorobutyle, trifluoroéthyle, 2-méthoxyéthyle ou 2-éthoxyéthyle.

12. Une composition selon la revendication 10, caractérisée en ce que $R^1$ est un radical méthyle, éthyle ou 2-méthoxyéthyle.

13. Un procédé de liaison de substrats poreux, caractérisé en ce qu'on applique une composition selon l'une quelconque des revendications 1 à 12 à au moins l'un des substrats et on joint les pendant un temps suffisant pour permettre à la composition de durcir.